# EUROPEAN PATENT SPECIFICATION

(11) **EP 4 059 412 B1**
(45) Date of publication and mention of the grant of the patent: **30.07.2025**
(21) Application number: 20904884.2
(22) Date of filing: 31.10.2020
(51) Int. Cl.: A61B 5/021, A61B 5/022

(54) **AIRBAG OF ELECTRONIC SPHYGMOMANOMETER, AND ELECTRONIC SPHYGMOMANOMETER**
AIRBAG FÜR ELEKTRONISCHES BLUTDRUCKMESSGERÄT UND ELEKTRONISCHES BLUTDRUCKMESSGERÄT
COUSSIN GONFLABLE DE SPHYGMOMANOMÈTRE ÉLECTRONIQUE, ET SPHYGMOMANOMÈTRE ÉLECTRONIQUE

(30) Priority: 23.12.2019 CN 201911341166
(43) Date of publication of application: 21.09.2022
(73) Proprietor: Huawei Technologies Co., Ltd., Shenzhen, Guangdong 518129 (CN)
(72) Inventor: FU, Xiaoyu, Shenzhen, Guangdong 518129 (CN); ZHOU, Linfeng, Shenzhen, Guangdong 518129 (CN); HUANG, Zhenlong, Shenzhen, Guangdong 518129 (CN); WU, Huangwei, Shenzhen, Guangdong 518129 (CN)
(74) Representative: Pfenning, Meinig & Partner mbB
(86) International application number: PCT/CN2020/125625
(87) International publication number: WO 2021/129153

(56) References cited:
- WO-A2-2012/040657
- CN-A- 105 686 817
- CN-A- 109 044 310
- CN-U- 208 677 378
- US-A1- 2003 055 347
- US-A1- 2006 135 873
- US-A1- 2018 353 347

## Description

### TECHNICAL FIELD

This application relates to the field of medical devices, and in particular, to an airbag of an electronic blood pressure monitor and an electronic blood pressure monitor.

### BACKGROUND

An electronic blood pressure monitor is a medical device that measures blood pressure based on an indirect blood pressure measurement principle by using electronic technologies. Product portability is improved because of miniaturization of the electronic blood pressure monitor, and therefore the electronic blood pressure monitor becomes suitable for household use and meets a daily requirement of a family for blood pressure measurement. However, blood pressure measurement accuracy of a miniaturized electronic blood pressure monitor is low.
US 2006/135873 A1 discloses a cuff for a blood pressure monitor that has an air bag inflated/deflated as the air comes in/out. The air bag has an inner wall portion located on the inner side in the fitted state of the cuff and an outer wall portion located outer than the inner wall portion, and a side wall portion connecting side end portions of the inner and outer wall portions and folded inwards in the deflated state of the air bag to form a gusset at each side end portion of the air bag. A bonded portion is provided at a region of the air bag in its winding direction around the living body, for reducing expansion of the gusset formed by the side wall portion. Thus, occurrence of lateral displacement of the cuff is prevented, and a highly reliable blood pressure monitor of high performance can be obtained.
US 2003/055347 A1 discloses a bladder incorporated in a cuff that includes an outer wall located at the outer side, an inner wall located at the inner side, side walls connected to both side ends of the outer wall and the inner wall in the wrapping direction, and folded inward of the bladder, and a joint connecting the side walls inside the bladder. A sphygmomanometer cuff is provided that can maintain the former configuration even when inflated or deflated without change in the width even if the bladder is inflated, and that does not dilate in the width direction when inflated.

### SUMMARY

This application provides an airbag of an electronic blood pressure monitor and an electronic blood pressure monitor, to improve blood pressure measurement accuracy. The invention is defined in the independent claims.

According to a first aspect, this application provides an airbag of an electronic blood pressure monitor. The airbag includes an outer wall and at least one connection wall, where the outer wall includes a first wall, a peripheral side wall, and a second wall; the first wall and the second wall are disposed opposite to each other; the peripheral side wall is connected to a periphery of the first wall and a periphery of the second wall; the first wall, the peripheral side wall, and the second wall form an air chamber; the at least one connection wall is located in the air chamber; each connection wall is connected to the peripheral side wall and the first wall or to the peripheral side wall and the second wall; and each connection wall is tensed when the airbag is in an expanded state.

The electronic device may include a processing module, an air pump, and a barometric pressure sensor. Both the air pump and the barometric pressure sensor communicate with the airbag. The air pump is configured to inflate the airbag, and the barometric pressure sensor is configured to collect barometric pressure of air in the airbag. A blood pressure measurement principle may be as follows. The air pump inflates the airbag, so that the airbag expands and compresses a blood vessel. When the airbag expands to a specific extent, the blood vessel is compressed by the airbag and then closes, so that a blood flow is blocked. When the blood flow is completely blocked, the airbag is controlled to deflate. In this case, the blood vessel generates a vibration waveform. The vibration waveform may cause air oscillation in the airbag, and an air oscillation waveform is related to the vibration waveform of the blood vessel. The barometric pressure sensor communicating with the airbag can collect an air oscillation waveform signal, and send the air oscillation waveform signal to the processing module. The processing module processes the oscillation waveform signal according to a built-in algorithm, and can calculate a blood pressure value.

In a natural state, the airbag may be approximately a flat strip-shaped bag, and the airbag may be wrapped around human skin to form an annular area with a specific width. The airbag has a length direction, a width direction, and a thickness direction. When the airbag is wrapped around the human skin, a contour of the airbag that originally extends along the length direction surrounds the human skin. The width direction is a width direction of the annular area. The thickness direction is a direction from the human skin to the airbag and an opposite direction. The airbag may be made of soft, deformable, and non-extendable materials.

The first wall and the second wall are respectively located at two opposite ends in the thickness direction. When the airbag is in use, the second wall may face the human skin, and the first wall may face away from the human skin. The peripheral side wall may be separated from the first wall or the second wall. When the airbag is formed, the peripheral side wall is "sewn" with the first wall and the second wall, and there is a "seam" between the peripheral side wall and each of the first wall and the second wall. Alternatively, the peripheral side wall may be connected to the first wall and the second wall, and the peripheral side wall smoothly transits to both the first wall and the second wall without "seams". There is at least one connection wall, and a single connection wall may be connected between the first wall and the second wall, between the first wall and the peripheral side wall, or between the second wall and the peripheral side wall, or connected to the first wall, the peripheral side wall, and the second wall simultaneously. Connection positions of different connection walls may be the same or different.

When the airbag is in the expanded state, the outer wall and all connection walls of the airbag are tensed. Because the connection wall can pull a corresponding outer wall of the airbag inward when the connection wall is tensed, outward protrusion of the corresponding outer wall is reduced or avoided, and an outline of an expanded airbag can be optimized. In this way, air in the airbag can be pressed more toward the second wall, pressure is more concentrated on the blood vessel, and an effective compression area of the airbag on the blood vessel is increased. As a result, a degree of compression-caused closure of the blood vessel is improved, and blood pressure measurement accuracy is further improved.

In an implementation, the peripheral side wall includes a first side wall and a second side wall that are opposite to each other; the at least one connection wall includes a first connection wall and a second connection wall; the first connection wall is connected to the first side wall and the first wall; one end of the second connection wall is connected to the second side wall; and an opposite end of the second connection wall is connected to the first wall or the second wall.

The first side wall and the second side wall may be respectively located at two opposite ends in the width direction, and both are connected to the periphery of the first wall and the periphery of the second wall. The first side wall and the second side wall may be respectively located at two opposite ends in the length direction. When the airbag is in the expanded state, because the first side wall is pulled by the first connection wall, outward protrusion of the first side wall is suppressed. Similarly, the second side wall is pulled by the second connection wall. Therefore, outward protrusion of the second side wall is suppressed. The first wall may be pulled by both the first connection wall and the second connection wall, and therefore outward protrusion of the first wall is suppressed; or the first wall is pulled by the first connection wall, and the second wall is pulled by the second connection wall, and therefore outward protrusion of the first wall and the second wall is suppressed. In this structure, air in the airbag can be pressed more toward the second wall, pressure is more concentrated on the blood vessel, and an effective compression area of the airbag on the blood vessel is increased. As a result, a degree of compression-caused closure of the blood vessel is improved, and blood pressure measurement accuracy is further improved. In addition, after the airbag is deflated, the first side wall at one end in the width direction (or the length direction) of the airbag is pulled inward by the first connection wall, and the second side wall at the other end in the width direction (or the length direction) of the airbag is pulled inward by the second connection wall, so that the first side wall and the second side wall do not extend. Therefore, the airbag does not squeeze outward, and product aesthetics and user experience are not affected.

In an implementation, the peripheral side wall includes a first side wall and a second side wall that are opposite to each other; the at least one connection wall includes a first connection wall and a second connection wall; the first connection wall is connected to the first side wall and the first wall; and the second connection wall is connected to the first side wall and the second wall. Because the first side wall, the first wall, and the second wall are pulled and in limited positions, blood pressure measurement accuracy can be improved. In addition, the first side wall does not extend after the airbag deflates, so that the airbag does not squeeze outward, and product aesthetics and user experience are not affected.

In an implementation, the at least one connection wall includes a third connection wall and a fourth connection wall; the third connection wall is connected to the first side wall and the second wall; and the fourth connection wall is connected to the second side wall and the second wall. By using the airbag structure, blood pressure measurement accuracy can be improved. In addition, the airbag does not extend after deflating, so that the airbag does not squeeze outward, and product aesthetics and user experience are not affected.

In an implementation, each connection wall has a first end, a middle part, and a second end, where the middle part is between the first end and the second end, the first end is connected to the first wall, the middle part is connected to the peripheral side wall, and the second end is connected to the second wall. The connection walls connect the first wall, the peripheral side wall, and the second wall simultaneously, so that the blood pressure measurement accuracy can be improved by using a simple structure, and a technical effect that the airbag does not extend after deflating can be ensured. The airbag of this kind is simple in design and easy to manufacture and produce massively.

In an implementation, each connection wall has pleats, and the pleats are in a stretched state when the connection wall is tensed. A pleat structure can provide a sufficient deformation margin, and switching of the first connection wall between a loosened state and a tensed state can be implemented.

In an implementation, the at least one connection wall and the outer wall form different chambers, and adjacent chambers communicate. This design allows air to flow between the chambers to ensure the normal inflation and deflation of the airbag, so that an operating need can be met.

In an implementation, a periphery of each connection wall is connected to the outer wall, each connection wall is provided with an air hole, and adjacent chambers communicate through the air hole. The periphery of each connection wall is connected to the outer wall of the airbag. This structure can enhance structure strength of the connection wall, and ensure pulling force of the connection wall. This helps optimize the outline of the inflated airbag, so that the blood pressure measurement accuracy can be improved. In this design, each connection wall partitions the air chamber. Therefore, the air hole is provided to implement interconnection between the chambers of the air chamber, and ensure normal inflation and deflation of the airbag.

In an implementation, each connection wall is connected to a middle part of the first wall or a middle part of the second wall. When the connection wall is connected to the first wall, the connection wall is connected to the middle part of the first wall. Alternatively, when the connection wall is connected to the second wall, the connection wall is connected to the middle part of the second wall. A center of the first wall or the second wall may be determined. An area between two sides of the center in the width direction may be referred to as a middle part of the first wall or the second wall in the width direction. The area between the two sides may be mirror-symmetric with the center being a point of symmetry. The connection wall is connected to the middle part of the first wall or the second wall, so that the pulling force may be exerted on a position that may be most protruding on the first wall or the second wall, to ensure that the first wall or the second wall generally does not protrude outward. In this way, air in the airbag can be pressed more toward the second wall, pressure is more concentrated on the blood vessel, and the effective compression area of the airbag on the blood vessel is increased. In this way, the degree of compression-caused closure of the blood vessel can be improved, and therefore the blood pressure measurement accuracy can be improved.

According to a second aspect, this application provides an electronic blood pressure monitor, including an air pump, a barometric pressure sensor, and an airbag, where the airbag includes an air nozzle connected to the air chamber, and both the air pump and the barometric pressure sensor communicate with the air chamber through the air nozzle. The air nozzle may be disposed on any of the foregoing outer walls. Because a collection wall of the airbag can pull an outer wall corresponding to the airbag inward when the collection wall is tensed, air in the airbag can be pressed more toward a second wall, pressure is more concentrated on a blood vessel, and an effective compression area of the airbag on the blood vessel is increased. As a result, a degree of compression-caused closure of the blood vessel is improved, and blood pressure measurement accuracy is improved by using the electronic blood pressure monitor having the airbag.

In an implementation, the air nozzle includes an air intake nozzle and a measurement air nozzle, the air pump communicates with the air chamber through the air intake nozzle, and the barometric pressure sensor communicates with the air chamber through the measurement air nozzle. The two air nozzles are disposed to perform inflation and measurement, so that it can be ensured that each system of the electronic blood pressure monitor operates without interfering with each other, and working reliability of the electronic blood pressure monitor is ensured.

In an implementation, the electronic blood pressure monitor is a sphygmomanometer, a wrist blood pressure monitor, or a wearable blood pressure monitor. An electronic blood pressure monitor of this kind is highly portable and suitable for household use.

### BRIEF DESCRIPTION OF DRAWINGS

FIG. 1 is a schematic diagram of a scenario in which a sphygmomanometer measures blood pressure according to an embodiment;
FIG. 2 is a schematic diagram of a scenario in which a wrist blood pressure monitor measures blood pressure according to another embodiment;
FIG. 3 is a schematic diagram of a three-dimensional structure of a blood pressure watch according to still another embodiment;
FIG. 4 is a schematic diagram of a three-dimensional sectional view of a structure of an airbag that is of an electronic blood pressure monitor and that is not inflated according to Embodiment 1;
FIG. 5 is a schematic diagram of a top view of the structure of the airbag in FIG. 4;
FIG. 6 is a schematic diagram of a side view of the structure of the airbag in FIG. 4;
FIG. 7 is a schematic diagram of a top view of a structure of an airbag according to yet another embodiment;
FIG. 8 is a schematic diagram of a top view of a structure of an airbag according to still yet another embodiment;
FIG. 9 is a schematic diagram of a three-dimensional sectional view of a structure of the airbag in FIG. 4 after the airbag is inflated and expands;
FIG. 10 is a schematic diagram of a side view of the structure of the airbag in FIG. 9;
FIG. 11 is a schematic diagram of a principle of compression by a conventional airbag on a blood vessel;
FIG. 12 is a schematic diagram of an outline change of a conventional airbag switching from an expanded state to a deflated state;
FIG. 13 is a schematic diagram of a principle of compression on a blood vessel by an airbag according to Embodiment 1;
FIG. 14 is a schematic diagram of a side view of a structure of an airbag that is not inflated according to Embodiment 2;
FIG. 15 is a schematic diagram of a principle of compression on a blood vessel by the airbag in FIG. 14 after the airbag is inflated and expands;
FIG. 16 is a schematic diagram of a side view of a structure of an airbag that is not inflated according to Embodiment 3;
FIG. 17 is a schematic diagram of a side view of a structure of the airbag in FIG. 16 after the airbag is inflated and expands;
FIG. 18 is a schematic diagram of a side view of a structure of an airbag that is not inflated according to Embodiment 4;
FIG. 19 is a schematic diagram of a side view of a structure of the airbag in FIG. 18 after the airbag is inflated and expands;
FIG. 20 is a schematic diagram of a three-dimensional sectional view of a structure of an airbag that is of an electronic blood pressure monitor and that is not inflated according to Embodiment 5;
FIG. 21 is a schematic diagram of a side view of the structure of the airbag in FIG. 20;
FIG. 22 is a schematic diagram of a three-dimensional sectional view of a structure of the airbag in FIG. 20 after the airbag is inflated and expands;
FIG. 23 is a schematic diagram of a side view of the structure of the airbag in FIG. 22;
FIG. 24 is a schematic diagram of a three-dimensional sectional view of a structure of an airbag that is of an electronic blood pressure monitor and that is not inflated according to Embodiment 6;
FIG. 25 is a schematic diagram of a top view of the structure of the airbag in FIG. 24;
FIG. 26 is a schematic diagram of a side view of the structure of the airbag in FIG. 24;
FIG. 27 is a schematic diagram of a three-dimensional sectional view of a structure of the airbag in FIG. 24 after the airbag is inflated and expands; and
FIG. 28 is a schematic diagram of a side view of the structure of the airbag in FIG. 27.

### DESCRIPTION OF EMBODIMENTS

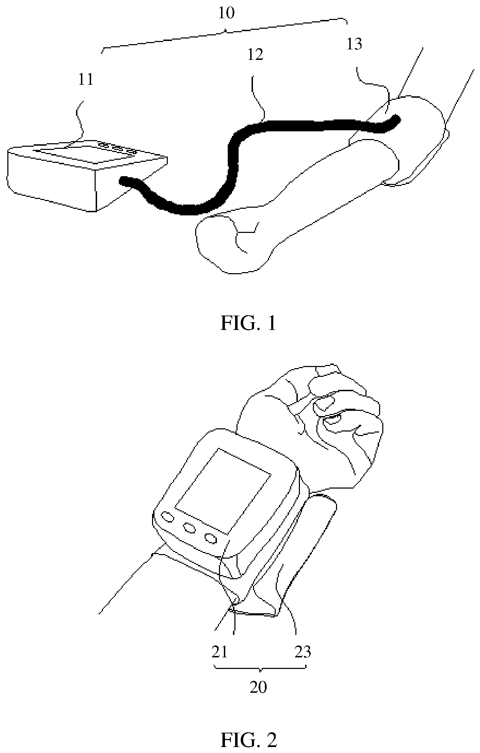

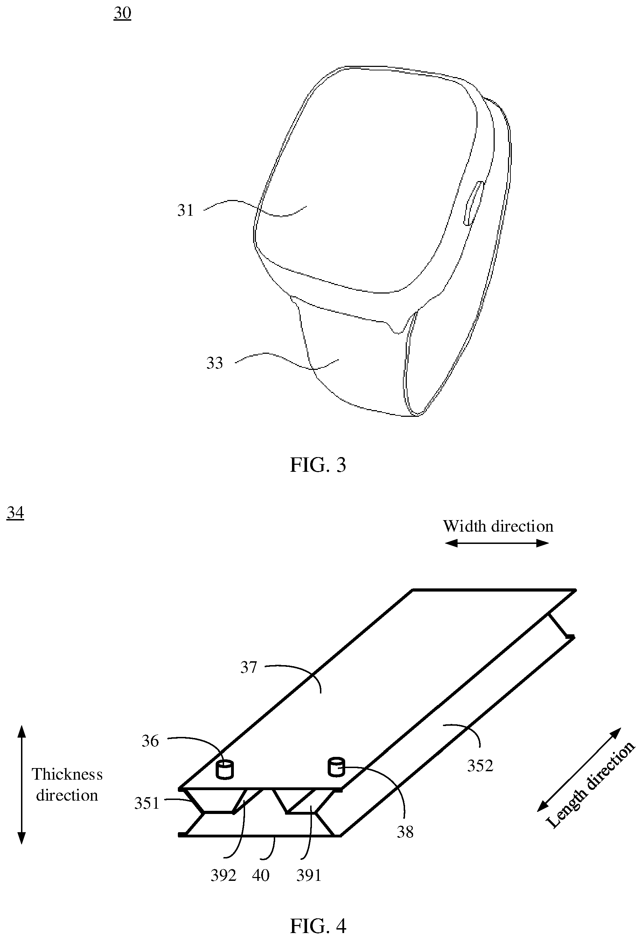

The following embodiment of this application provides an electronic blood pressure monitor, including but not limited to including a sphygmomanometer 10 shown in FIG. 1, a wrist blood pressure monitor 20 shown in FIG. 2, or a blood pressure watch 30 shown in FIG. 3. Specific descriptions are provided below.

As shown in FIG. 1, the sphygmomanometer 10 may include a main unit 11, a hose 12, and a cuff 13, where the hose 12 is connected to the main unit 11 and the cuff 13. The main unit 11 may include a processing module, a display screen, an air pump, and a barometric pressure sensor. The cuff 13 can be wrapped around and bound to an arm of a human body, and an airbag is packaged in the cuff 13. An air pump may inflate the airbag through the hose 12, so that the airbag expands and compresses a blood vessel. When the airbag expands to a specific extent, the blood vessel is compressed by the airbag and then closes, so that a blood flow is blocked. When the blood flow is completely blocked, the airbag is controlled to deflate. In this case, the blood vessel generates a vibration waveform. The vibration waveform may cause air oscillation in the airbag, and an air oscillation waveform is related to the vibration waveform of the blood vessel. The barometric pressure sensor communicating with the airbag can collect an air oscillation waveform signal, and send the air oscillation waveform signal to the processing module. The processing module processes the oscillation waveform signal according to a built-in algorithm (for example, the built-in algorithm includes an amplitude coefficient method), and can calculate a blood pressure value. The processing module can further control the display screen to display the blood pressure value.

FIG. 2 schematically shows a main unit 21 and a wrist band 23 of a wrist blood pressure monitor 20. The wrist band 23 can be wrapped around and bound to a wrist of a human body, and an airbag is packaged in the wrist band 23. The main unit 21 may include a processing module, a display screen, an air pump, and a barometric pressure sensor. Both the air pump and the barometric pressure sensor communicate with the airbag. A blood pressure measurement principle of the wrist blood pressure monitor 20 is the same as that described above, and details are not described herein again.

FIG. 3 schematically shows a main unit 31 and a watch band 33 of a blood pressure watch 30. The watch band 33 can be wrapped around and bound to a wrist of a human body, and an airbag is packaged in the watch band 33. The main unit 31 may include a processing module, a display screen, an air pump, and a barometric pressure sensor. Both the air pump and the barometric pressure sensor communicate with the airbag. A blood pressure measurement principle of the blood pressure watch 30 is the same as that described above, and details are not described herein again. In another embodiment, in addition to the blood pressure watch 30, the electronic blood pressure monitor may alternatively be another wearable blood pressure monitor (a portable blood pressure monitor suitable for long-term wearing), for example, a blood pressure wristband.

The following describes in detail a structure of the airbag of the electronic device.

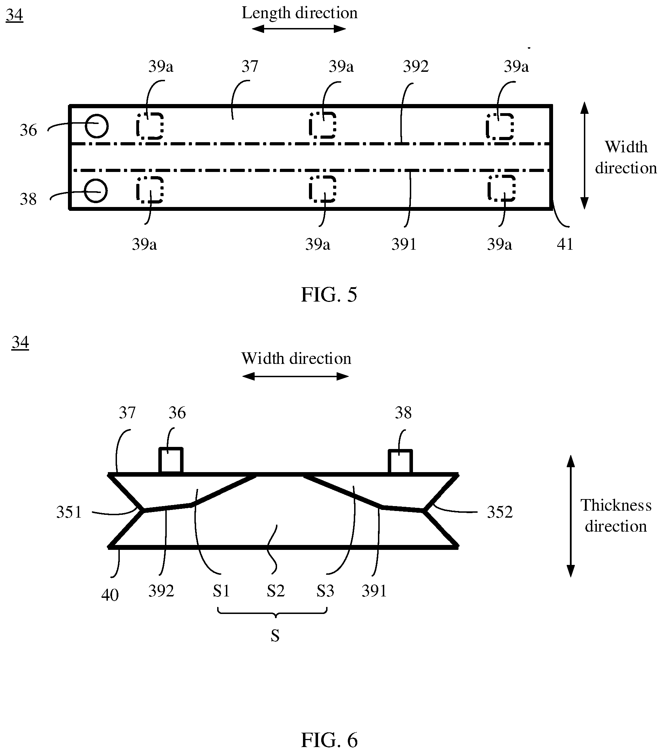

As shown in FIG. 4, in Embodiment 1, in a natural state, the airbag 34 may be approximately a flat strip-shaped bag, and the airbag 34 may be wrapped around an arm or a wrist to form an annular area with a specific width. The airbag 34 has a length direction, a width direction, and a thickness direction. When the airbag 34 is wrapped around the arm or the wrist, a contour of the airbag 34 that originally extends along the length direction surrounds the arm or the wrist. The width direction is a width direction of the annular area. The thickness direction is a direction from human skin to the airbag 34 and an opposite direction (for example, from a perspective shown in FIG. 6, the thickness direction is a vertical direction). The airbag may be made of soft, deformable, and non-extendable materials, such as thermoplastic polyurethane (TPU) or polyvinyl chloride (PVC).

As shown in FIG. 4 to FIG. 6, the airbag 34 has an outer wall, and the outer wall may include a first wall 37, a second wall 40, a first side wall 351, and a second side wall 352. The airbag 34 may further include a first connection wall 392 and a second connection wall 391.

The first wall 37 and the second wall 40 are disposed opposite to each other, and the first wall 37 and the second wall 40 are respectively located at two opposite ends in the thickness direction. When the airbag 34 is in use, the second wall 40 may face human skin, and the first wall 37 may face away from human skin. Shapes of the first wall 37 and the second wall 40 are not limited. For example, the first wall 37 and the second wall 40 may be approximately in a shape of a flat strip. An air intake nozzle 36 and a measurement air nozzle 38 may be disposed on the first wall 37. An air pump communicates with the air intake nozzle 36, and a barometric pressure sensor communicates with the measurement air nozzle 38.

In another embodiment, the air intake nozzle 36 and the measurement air nozzle 38 are independently disposed, and respective positions of the air intake nozzle 36 and the measurement air nozzle 38 may be determined based on a requirement for the product. Positions of the air intake nozzle 36 and the measurement air nozzle 38 are not limited to a same wall. For example, one of the air intake nozzle 36 and the measurement air nozzle 38 may be disposed on the first side wall 351 described below, and the other is disposed on the second side wall 352 described below; or both the air intake nozzle 36 and the measurement air nozzle 38 are disposed on the first side wall 351 or the second side wall 352. Alternatively, only one air nozzle may be disposed on the first wall 37, and both the air pump and the barometric pressure sensor communicate with the air nozzle. Alternatively, the air nozzle may be disposed in another position, for example, a peripheral side wall described below. The barometric pressure sensor may alternatively be disposed at the measurement air nozzle 38 or the air nozzle, instead of being included in the main unit.

The first side wall 351 and the second side wall 352 are disposed opposite to each other, and the first side wall 351 and the second side wall 352 are located at two opposite ends in the width direction. Both the first side wall 351 and the second side wall 352 are connected to a periphery of the first wall 37 and a periphery of the second wall 40.

As shown in FIG. 5, the airbag 34 may further include a side wall 41 located at one end in the length direction, and a side wall (in FIG. 5, the side wall is cut off from a sectional perspective and is therefore not shown) that is opposite to the side wall 41 and located at the other end in the length direction. Both the side wall 41 and the side wall are connected to the periphery of the first wall 37 and the periphery of the second wall 40. The side wall 41, the first side wall 351, the side wall, and the second side wall 352 are connected to form a peripheral side wall of the airbag 34. The peripheral side wall may be separated from the first wall 37 or the second wall 40. When the airbag 34 is formed, the peripheral side wall is "sewn" with the first wall 37 and the second wall 40, and there is a "seam" between the peripheral side wall and each of the first wall 37 and the second wall 40. Alternatively, the peripheral side wall may be connected to the first wall 37 and the second wall 40, and the peripheral side wall smoothly transits to both the first wall 37 and the second wall 40 without "seams".

Refer to FIG. 4 and FIG. 6. The first wall 37, the peripheral side wall, and the second wall 40 form an air chamber S. The air intake nozzle 36 and the air chamber S communicate, so that the air pump can inflate the air chamber S. The measurement air nozzle 38 and the air chamber S also communicate, so that the barometric pressure sensor can collect an air oscillation waveform signal in the airbag 34.

As shown in FIG. 4 to FIG. 6, the first connection wall 392 may be approximately in a shape of a flat strip. The first connection wall 392 is located in the air chamber S, and is connected between the first side wall 351 and the first wall 37. Two opposite ends of the first connection wall 392 in the length direction are also respectively connected to the side wall 41 and the side wall. In other words, a periphery of the first connection wall 392 is connected to the outer wall of the airbag 34.

A connection position of the first connection wall 392 on each outer wall may be in a middle part of a corresponding outer wall. As shown in FIG. 4 and FIG. 6, the first connection wall 392 may be connected to a middle part of the first side wall 351 in the thickness direction. A center of the first side wall 351 may be determined. An area between two sides of the center in the thickness direction may be referred to as a middle part in the thickness direction. The area between the two sides may be mirror-symmetric with the center being a point of symmetry, and a width of an area on each side may be set based on a requirement. The first connection wall 392 may be connected to a middle part of the first wall 37 in the width direction. Similarly, a center of the first wall 37 may be determined. An area between two sides of the center in the width direction may be referred to as a middle part in the width direction. The area between the two sides may be mirror-symmetric with the center being a point of symmetry, and a width of an area on each side may be set based on a requirement. In another embodiment, the connection position of the first connection wall 392 on each outer wall may be determined according to a requirement for the product, and is not limited to a middle part of a corresponding outer wall.

The periphery of the first connection wall 392 is connected to an outer wall of the airbag 34, to divide the air chamber S into different chambers. For example, as shown in FIG. 6, the first connection wall 392 may divide a chamber S1 from the air chamber S.

As shown in FIG. 4 to FIG. 6, the second connection wall 391 may be approximately in a shape of a flat strip. The second connection wall 391 is located in the air chamber S, and is connected between the second side wall 352 and the first wall 37. Two opposite ends of the second connection wall 391 in the length direction are also respectively connected to the side wall 41 and the side wall. In other words, a periphery of the second connection wall 391 is connected to the outer wall of the airbag 34.

A connection position of the second connection wall 391 on each outer wall may be in a middle part of a corresponding outer wall. For example, as shown in FIG. 6, the second connection wall 391 may be connected to a middle part of the second side wall 352 in the thickness direction, and the second connection wall 391 may be connected to a middle part of the first wall 37 in the width direction. In another embodiment, the connection position of the second connection wall 391 on each outer wall may be determined according to a requirement for the product, and is not limited to a middle part of a corresponding outer wall.

The periphery of the second connection wall 391 is connected to an outer wall of the airbag 34, to divide the air chamber S into different chambers. For example, as shown in FIG. 6, the second connection wall 391 may divide a chamber S3 from the air chamber S, and the second connection wall 391 and the first connection wall 392 may divide a chamber S2 from the air chamber S. In other words, the air chamber S may be divided into the chamber S1, the chamber S2, and the chamber S3 through the design of the first connection wall 392 and the second connection wall 391.

Refer to FIG. 4 to FIG. 6. To achieve communication between the different chambers, several air holes 39a may be provided on both the first connection wall 392 and the second connection wall 391, so that the airbag 34 can completely expand during inflation. The air holes 39a are through holes. The air hole 39a on the first connection wall 392 is used to achieve communication between the chamber S1 and the chamber S2, and the air hole 39a on the second connection wall 391 is used to achieve communication between the chamber S2 and the chamber S3. A shape and a quantity of the air hole 39a is not limited, and may be designed according to a requirement for the product.

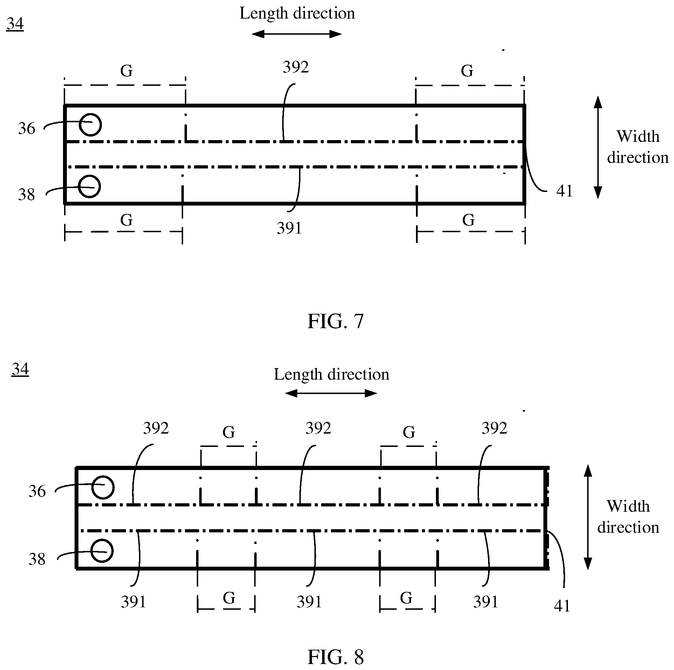

Refer to FIG. 4 and FIG. 7. In another embodiment, a difference from Embodiment 1 is that the first connection wall 392 is connected between the first wall 37 and the first side wall 351, but both opposite ends of the first connection wall 392 in the length direction are not connected to the peripheral side wall of the airbag 34, and there is a spacing G between the peripheral side wall and each of the opposite ends of the first connection wall 392. For example, as shown in FIG. 7, a right end of the first connection wall 392 in the length direction is not connected to the side wall 41 of the peripheral side wall, and there is a spacing G between the right end and the side wall 41. In other words, only a part of the periphery of the first connection wall 392 is connected to the outer wall of the airbag 34. Because the first connection wall 392 does not partition the air chamber in this case, the air hole may not be provided on the first connection wall 392.

Refer to FIG. 4 and FIG. 7. In another embodiment, the second connection wall 391 is connected between the first wall 37 and the second side wall 352, but both opposite ends of the second connection wall 391 in the length direction are not connected to the sides of the airbag 34, and there is a spacing G between the peripheral side wall and each of the opposite ends of the second connection wall 391. For example, as shown in FIG. 7, a right end of the second connection wall 391 in the length direction is not connected to the side wall 41 of the peripheral side wall, and there is a spacing G between the right end and the side wall 41. In other words, only a part of the periphery of the second connection wall 391 is connected to the outer wall of the airbag 34. Similarly, the air hole may not be provided on the second connection wall 391.

Alternatively, in another embodiment, as shown in FIG. 8, there may be several (for example, three) first connection walls 392, the several first connection walls 392 are disposed side by side, and there is a spacing G between neighboring first connection walls 392. Each first connection wall 392 is connected between the first wall 37 and the first side wall 351, but at least one end in the length direction is not connected to the peripheral side wall of the airbag 34. For example, the right end of a first connection wall 392 that is at the left end is not connected to the peripheral side wall of the airbag 34, the left end of a first connection wall 392 that is at the right end is not connected to the peripheral side wall of the airbag 34, and neither the left end nor the right end of a first connection wall 392 at the middle is connected to the peripheral side wall of the airbag 34. Certainly, FIG. 8 is merely an example. For example, neither of opposite ends of each first connection wall 392 may be connected to the peripheral side wall of the airbag 34. Because the first connection walls 392 do not partition the air chamber in this case, air holes may not be provided on the first connection walls 392.

In another embodiment, as shown in FIG. 8, there may also be several (for example, three) second connection walls 391, the several second connection walls 391 are disposed side by side, and there is a spacing G between neighboring second connection walls 391. Each second connection wall 391 is connected between the first wall 37 and the second side wall 352, but at least one end in the length direction is not connected to the peripheral side wall of the airbag 34. For example, the right end of a second connection wall 391 that is at the left end is not connected to the peripheral side wall of the airbag 34, the left end of a second connection wall 391 that is at the right end is not connected to the peripheral side wall of the airbag 34, and neither the left end nor the right end of a second connection wall 391 at the middle is connected to the peripheral side wall of the airbag 34. Certainly, FIG. 8 is merely an example. For example, neither of opposite ends of each second connection wall 391 may be connected to the peripheral side wall of the airbag 34. Because the second connection walls 391 do not partition the air chamber in this case, air holes may not be provided on the first connection walls 392.

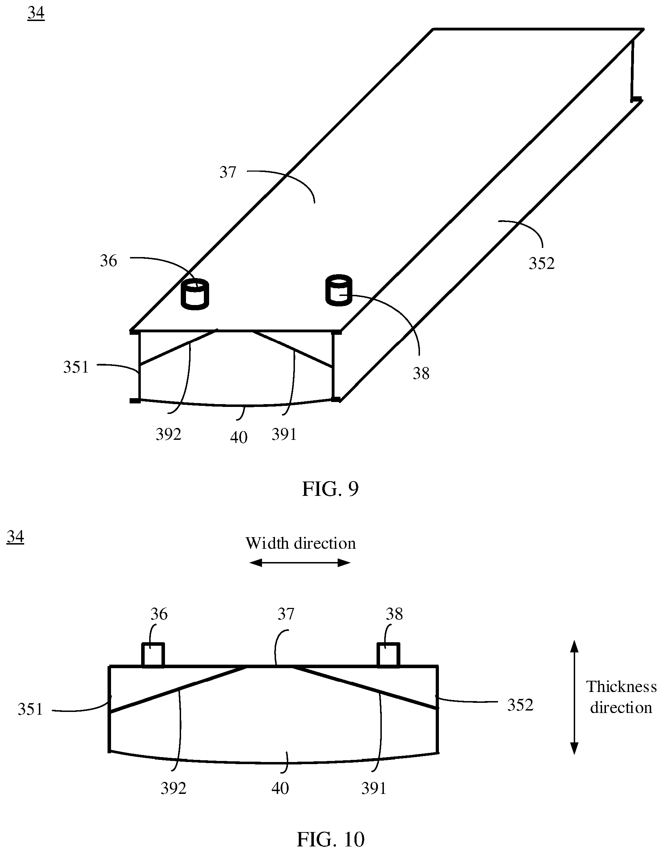

The airbag 34 in FIG. 4 to FIG. 8 is not inflated, and the foregoing outer walls, the first connection wall 392, and the second connection wall 391 of the airbag 34 are all in a deflated state. As shown in FIG. 9 and FIG. 10, during inflation of the airbag 34, the foregoing outer walls, the first connection wall 392, and the second connection wall 391 are all tensed. Being tensed is a state in which a shape is formed through tensing, and bending or deformation cannot be easily performed. Because the first side wall 351 is pulled by the first connection wall 392, and a connection position of the first connection wall 392 on the first side wall 351 is in the middle of the first side wall 351, the first side wall 351 generally does not protrude outward. Similarly, because the second side wall 352 is pulled by the second connection wall 391, and a connection position of the second connection wall 391 on the second side wall 352 is in the middle of the second side wall 352, the second side wall 352 generally does not protrude outward. The first wall 37 is pulled by both the first connection wall 392 and the second connection wall 391, and a connection position of the first connection wall 392 on the first wall 37 and a connection position of the second connection wall 391 on the first wall 37 are in the middle of the first wall 37. Therefore, the first wall 37 generally does not protrude outward. Because the second wall 40 is not pulled, the second wall 40 may slightly protrude outward.

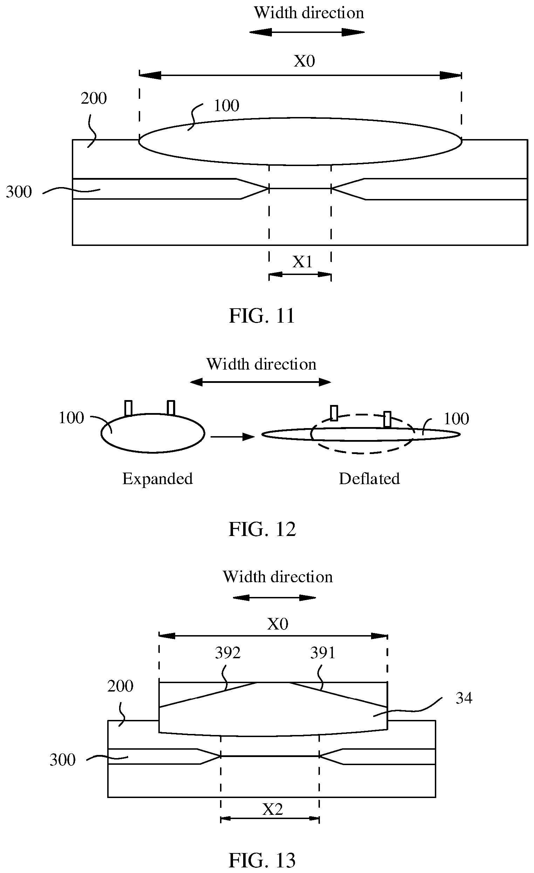

FIG. 11 is a schematic diagram of a principle of compressing a blood vessel 300 in human tissue 200 when a conventional airbag 100 expands. Because the conventional airbag 100 has only an outer wall, when in an expanded state, the conventional airbag 100 forms an elliptical cross section, where a lower wall in contact with human skin protrudes outward and forms an arc. Such a geometric structure causes an effective compression length X1 of the blood vessel 300 (a length of a closed part of the blood vessel 300 under compression) to be much less than a width X0 of the conventional airbag 100. Consequently, pressure exerted by the conventional airbag 100 on the blood vessel 300 is limited, and the blood vessel 300 cannot be completely compressed and closed. Therefore, blood pressure measurement accuracy is reduced.

In addition, as shown in FIG. 12, after the conventional airbag 100 is deflated, two opposite ends of the conventional airbag 100 in the width direction loosen and extend. As a result, the conventional airbag 100 squeezes a cuff, a wristband, or a watchband, which not only affects appearance of the product, but also causes user discomfort.

On the contrary, as shown in FIG. 13, because the airbag 34 in Embodiment 1 has the first connection wall 392 and the second connection wall 391, an outline of an expanded airbag 34 can be optimized, and air in the airbag 34 is limited to flow to the first wall 37, the first side wall 351, and the second side wall 352, so that the air can be pressed more toward the second wall 40, and pressure is concentrated more on the blood vessel 300. It can be learned by comparing FIG. 13 with FIG. 11 that an effective compression length X2 of the blood vessel 300 in FIG. 13 is greater than the effective compression length X1 in FIG. 11. Because the pressure exerted by the airbag 34 on the blood vessel 300 is increased, a degree of compression-caused closure of the blood vessel 300 can be improved. In this case, the blood pressure measurement accuracy can be improved. In addition, as shown in FIG. 6, after the airbag 34 in Embodiment 1 is deflated, the first side wall 351 at one end in the width direction of the airbag 34 is pulled inward by the first connection wall 392, and the second side wall 352 at the other end in the width direction of the airbag 34 is pulled inward by the second connection wall 391, so that the first side wall 351 and the second side wall 352 do not extend. Therefore, the airbag 34 does not squeeze the cuff, the wristband, or the watchband, and product aesthetics and user experience are not affected.

In another embodiment, the first connection wall 392 may be connected to the first side wall 351 and the second wall 40, and/or the second connection wall 391 is connected to the second side wall 352 and the second wall 40. Alternatively, either the first connection wall 392 or the second connection wall 391 may exist, and the first connection wall 392 or the second connection wall 391 may be connected to any two outer walls of the airbag.

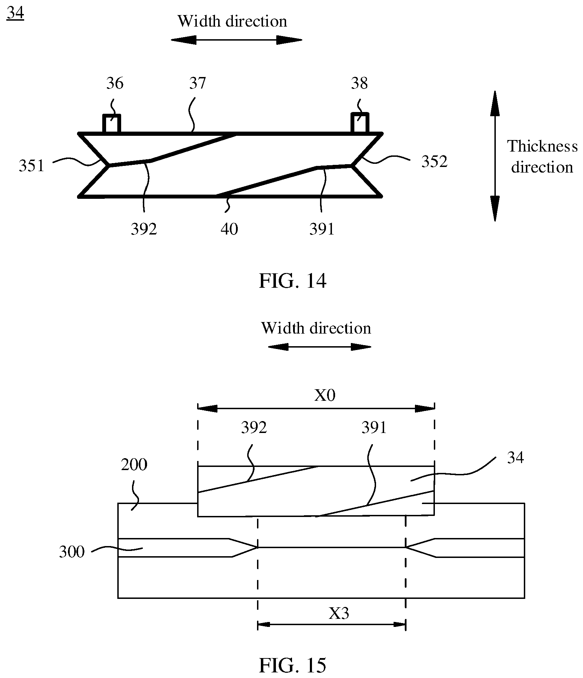

As shown in FIG. 14 and FIG. 15, in Embodiment 2, a difference from the foregoing Embodiment 1 is that one end of the second connection wall 391 is connected to the second side wall 352, and the other end is connected to the second wall 40. Because the second wall 40 is also pulled by the second connection wall 391, outward protrusion of the second wall 40 during inflation of the airbag 34 can be suppressed, and the second wall 40 becomes flatter. In this case, an effective compression length X3 of the blood vessel 300 is increased (the effective compression length X3 may be greater than the foregoing effective compression length X2), and a compression area of the airbag 34 is increased. Therefore, the pressure exerted by the airbag 34 on the blood vessel 300 is further increased, the degree of compression-caused closure of the blood vessel 300 can be improved. In this case, the blood pressure measurement accuracy can be improved.

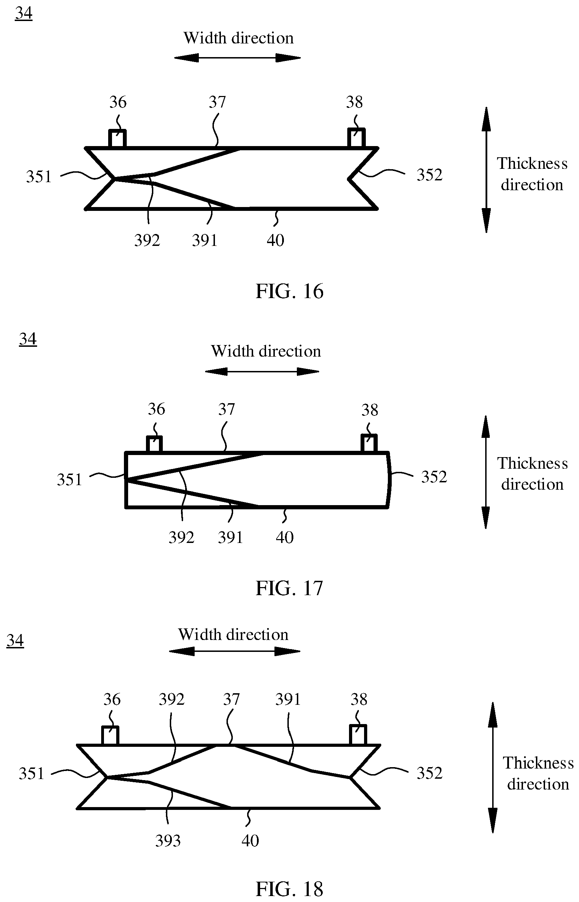

As shown in FIG. 16 and FIG. 17, in Embodiment 3, a difference from the foregoing Embodiment 1 is that the first connection wall 392 is connected between the first wall 37 and the first side wall 351, and the second connection wall 391 is connected between the first side wall 351 and the second wall 40. One end of the first connection wall 392 and one end of the second connection wall 391 may intersect on the first side wall 351, or may be separated from each other. Because the first wall 37, the first side wall 351, and the second wall 40 are all pulled and in limited positions, outward protrusion of the first wall 37, the first side wall 351, and the second wall 40 is suppressed when the airbag 34 expands. The second side wall 352 is not connected to the first connection wall 392 and the second connection wall 391, and the second side wall 352 may slightly protrude outward. However, according to the foregoing principle, generally, air is still pressed more toward the second wall 40 in the airbag 34, so that pressure is more concentrated on the blood vessel, and the degree of compression-caused closure of the blood vessel can be improved. In this way, the blood pressure measurement accuracy is improved. In addition, the first side wall 351 does not loosen and extend during deflation of the airbag 34. Therefore, the first side wall 351 does not squeeze the cuff, the wristband, or the watchband, and product aesthetics is not affected.

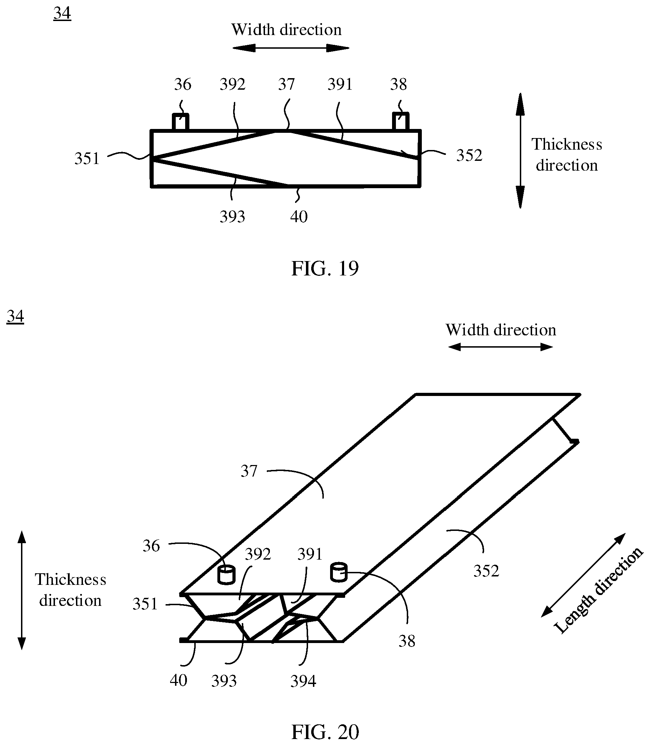

As shown in FIG. 18 and FIG. 19, in Embodiment 4, based on the solution of Embodiment 1, the airbag 34 may further include a third connection wall 393. The third connection wall 393 is connected between the first side wall 351 and the second wall 40. One end of the third connection wall 393 and one end of the first connection wall 392 may intersect on the first side wall 351, or may be separated from each other. Because the first wall 37, the second side wall 352, the second wall 40, and the first side wall 351 are all pulled and in limited positions, the outline of the expanded airbag 34 is further optimized, so that air can be pressed more toward the second wall 40, and pressure is more concentrated on the blood vessel. This further improves the degree of compression-caused closure of the blood vessel and further improves the blood pressure measurement accuracy. In addition, during deflation of the airbag 34, the first side wall 351 and the second side wall 352 do not extend. Therefore, the airbag 34 does not squeeze the cuff, the wristband, or the watchband, and product aesthetics and user experience are ensured.

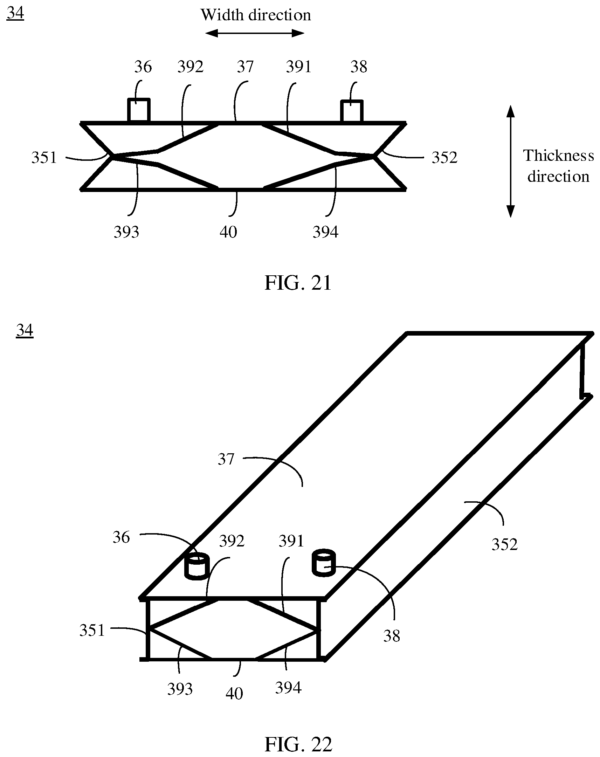

As shown in FIG. 20 and FIG. 21, in Embodiment 5, based on the solution of Embodiment 4, the airbag 34 may further include a fourth connection wall 394. The fourth connection wall 394 is connected between the second side wall 352 and the second wall 40. One end of the fourth connection wall 394 and one end of the second connection wall 391 may intersect on the second side wall 352, or may be separated from each other. The other end of the fourth connection wall 394 and one end of the third connection wall 393 may be separated on the second wall 40, or may intersect.

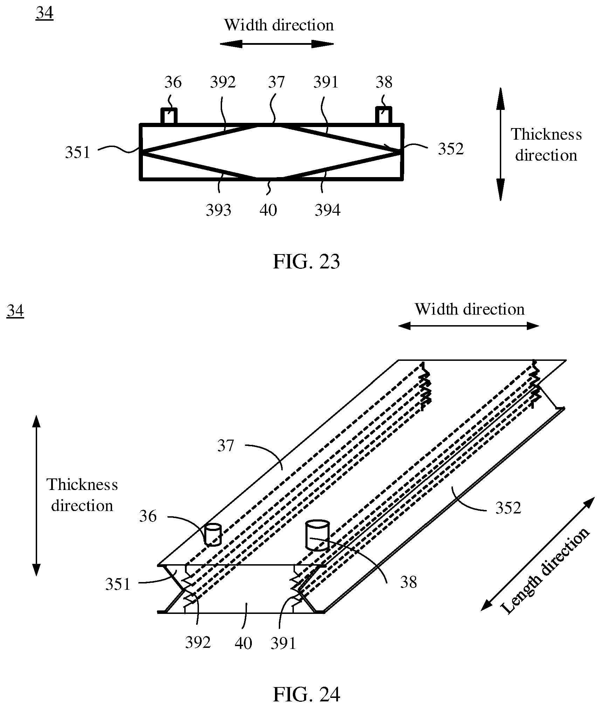

As shown in FIG. 22 and FIG. 23, when the airbag 34 is inflated and expands, because the first wall 37, the second side wall 352, the second wall 40, and the first side wall 351 are all pulled and in limited positions, the outline of the expanded airbag 34 is highly optimized, so that air can be pressed more toward the second wall 40, and pressure is more concentrated on the blood vessel. This further improves the degree of compression-caused closure of the blood vessel and further improves the blood pressure measurement accuracy. In addition, during deflation of the airbag 34, the first side wall 351 and the second side wall 352 do not extend. Therefore, the airbag 34 does not squeeze the cuff, the wristband, or the watchband, and product aesthetics and user experience are ensured.

As shown in FIG. 24 to FIG. 26, in Embodiment 6, a difference from the foregoing embodiments is that the first connection wall 392 of the airbag 34 connects the first wall 37, the first side wall 351, and the second wall 40, and the second connection wall 391 of the airbag 34 connects the first wall 37, the second side wall 352, and the second wall 40. Details are as follows.

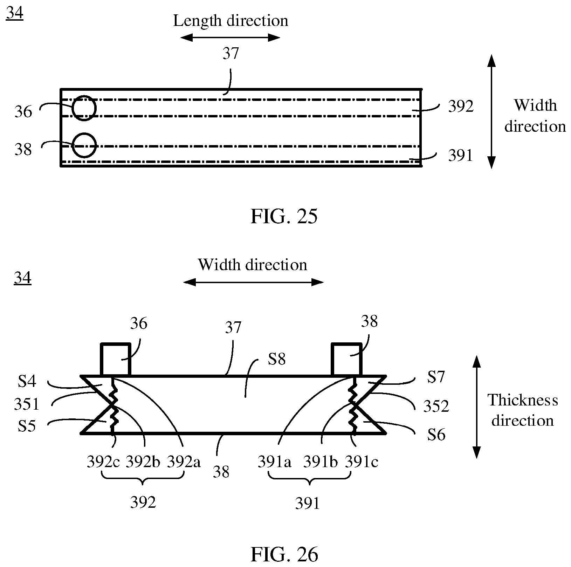

As shown in FIG. 26, the first connection wall 392 may have several pleats (similar to a leaf of a folding fan). The first connection wall 392 may have a first end 392a, a middle part 392b, and a second end 392c, and the middle part 392b is connected between the first end 392a and the second end 392c. The first end 392a is connected to the first wall 37, the middle part 392b is connected to the first side wall 351, and the second end 392c is connected to the second wall 40. The second connection wall 391 may also have several pleats. The second connection wall 391 may have a first end 391a, a middle part 391b, and a second end 391c, and the middle part 391b is connected between the first end 391a and the second end 391c. The first end 391a is connected to the first wall 37, the middle part 391b is connected to the second side wall 352, and the second end 391c is connected to the second wall 40. In other embodiments, at least one of the first connection wall 392 and the second wall 40 may connect only the first wall 37 and the second wall 40.

As shown in FIG. 26, the first connection wall 392 and the second connection wall 391 are disposed in the air chamber of the airbag 34, so that the air chamber can be divided into a chamber S4, a chamber S5, a chamber S6, a chamber S7, and a chamber S8. To achieve communication between the chamber S4 and the chamber S8, air holes may be provided in an area of the first connection wall 392 between the first end 392a and the middle part 392b, an area of the first connection wall 392 between the middle part 392b and the second end 392c, an area of the second connection wall 391 between the first end 391a and the middle part 391b, and an area of the second connection wall 391 between the middle part 391b and the second end 391c.

As described above, in another embodiment, there may be one first connection wall 392, or there may be several first connection walls 392 arranged side by side at intervals. At least one end of a single first connection wall 392 in the length direction may be spaced away from the peripheral side wall of the airbag 34. In other words, at least a part of a periphery of the first connection wall 392 is not connected to the outer wall of the airbag 34. Because the first connection wall 392 does not partition the air chamber in this case, the air holes may not be provided on the first connection wall 392. The second connection wall 391 may have a design similar to that of the first connection wall 392, and similarly, air holes may not be disposed on the second connection wall 391.

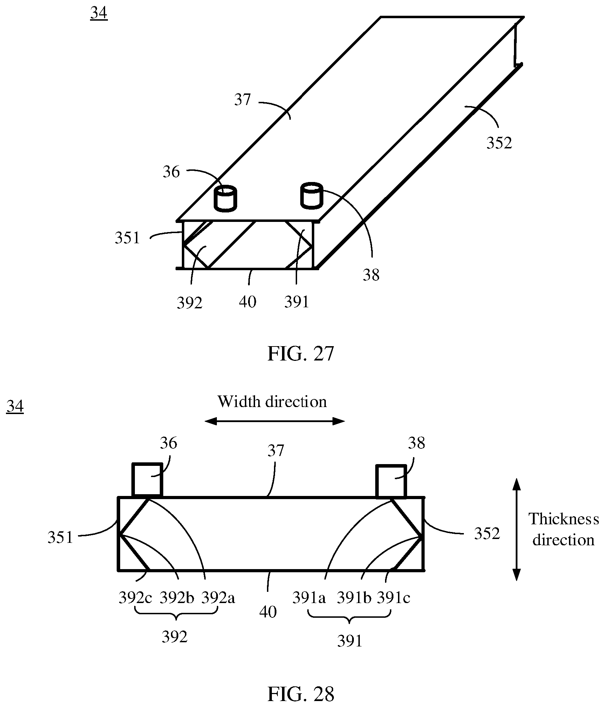

As shown in FIG. 27 and FIG. 28, when the airbag 34 is inflated and expands, pleats on the first connection wall 392 and the second connection wall 391 stretch, so that the first connection wall 392 and the second connection wall 391 are tensed. A pleat structure can provide a sufficient deformation margin, and switching of the first connection wall 392 between a loosened state and a tensed state can be implemented. In other embodiments, the pleats are not mandatory. For example, when the airbag 34 is deflated, the first connection wall 392 and the second connection wall 391 may naturally curl up.

Because the first wall 37, the second side wall 352, the second wall 40, and the first side wall 351 are all pulled and in limited positions, the outline of the expanded airbag 34 is highly optimized, so that air can be pressed more toward the second wall 40, and pressure is more concentrated on the blood vessel 300. This further improves the degree of compression-caused closure of the blood vessel 300 and further improves the blood pressure measurement accuracy. In addition, during deflation of the airbag 34, the first side wall 351 and the second side wall 352 do not extend. Therefore, the airbag 34 does not squeeze the cuff, the wristband, or the watchband, and product aesthetics and user experience are ensured.

## Claims

1. An airbag (34) of an electronic blood pressure monitor, wherein
the airbag (34) comprises an outer wall and at least one connection wall (391, 392, 393, 394); the outer wall comprises a first wall (37), a peripheral side wall, and a second wall (40); the first wall (37) and the second wall (40) are disposed opposite to each other; the peripheral side wall is connected to a periphery of the first wall (37) and a periphery of the second wall (40); the first wall (37), the peripheral side wall, and the second wall (40) form an air chamber (S);
**characterized in that** the at least one connection wall (391, 392, 393, 394) is located in the air chamber (S); each connection wall (391, 392, 393, 394) is connected to the peripheral side wall and the first wall (37) or to the peripheral side wall and the second wall (40); and each connection wall (391, 392, 393, 394) is configured to be tensed when the airbag (34) is in an expanded state.

2. The airbag (34) according to claim 1, wherein
the peripheral side wall comprises a first side wall (351) and a second side wall (352) that are opposite to each other; the at least one connection wall (391, 392, 393, 394) comprises a first connection wall (392) and a second connection wall (391); the first connection wall (392) is connected to the first side wall (351) and the first wall (37); one end of the second connection wall (391) is connected to the second side wall (352); and an opposite end of the second connection wall (391) is connected to the first wall (37) or the second wall (40).

3. The airbag (34) according to claim 1,
wherein the peripheral side wall comprises a first side wall (351) and a second side wall (352) that are opposite to each other; the at least one connection wall (391, 392, 393, 394) comprises a first connection wall (392) and a second connection wall (391); the first connection wall (392) is connected to the first side wall (351) and the first wall (37); and the second connection wall (391) is connected to the second side wall (352) and the first wall (37).

4. The airbag (34) according to claim 3, wherein
the at least one connection wall (391, 392, 393, 394) comprises a third connection wall (393) and a fourth connection wall (394); the third connection wall (393) is connected to the first side wall (351) and the second wall (40); and the fourth connection wall (394) is connected to the second side wall (352) and the second wall (40).

5. The airbag (34) according to claim 1, wherein
each connection wall (391, 392, 393, 394) has a first end (391a, 392a), a middle part (391b, 392b), and a second end (391c, 392c), wherein the middle part (391b, 392b) is between the first end (391a, 392a) and the second end (391c, 392c); the first end (391a, 392a) is connected to the first wall (37); the middle part (391b, 392b) is connected to the peripheral sidewall; and the second end (391c, 392c) is connected to the second wall (40).

6. The airbag (34) according to claim 5, wherein each connection wall (391, 392, 393, 394) has pleats, and the pleats are configured to be in a stretched state when the connection wall (391, 392, 393, 394) is tensed.

7. The airbag (34) according to any one of claims 1 to 6, wherein the at least one connection wall (391, 392, 393, 394) and the outer wall form different chambers (S1-S3), and adjacent chambers (S1-S3) communicate.

8. The airbag (34) according to claim 7, wherein a periphery of each connection wall (391, 392, 393, 394) is connected to the outer wall; each connection wall (391, 392, 393, 394) is provided with an air hole (39a); and adjacent chambers (S1-S3) communicate through the air hole (39a).

9. The airbag (34) according to any one of claims 1 to 8, wherein
each connection wall (391, 392, 393, 394) is connected to a middle part of the first wall (37) or a middle part of the second wall (40).

10. An electronic blood pressure monitor, comprising:
an air pump;
a barometric pressure sensor; and
the airbag (34) according to any one of claims 1 to 9, wherein the airbag (34) comprises an air nozzle (38)connected with an air chamber (S), and both the air pump and the barometric pressure sensor communicate with the air chamber (S) through the air nozzle (38).

11. The electronic blood pressure monitor according to claim 10, wherein the air nozzle (38) comprises an air intake nozzle (36) and a measurement air nozzle (38), the air pump communicates with the air chamber (S) through the air intake nozzle (36), and the barometric pressure sensor communicates with the air chamber (S) through the measurement air nozzle (38).

12. The electronic blood pressure monitor according to claim 10 or 11, wherein the electronic blood pressure monitor is a sphygmomanometer (10), a wrist blood pressure monitor (20), or a wearable blood pressure monitor (30).

13. The electronic blood pressure monitor according to claim 10 or 11, wherein the electronic blood pressure monitor comprises:
a main unit (11, 21, 31), the main unit (11, 21, 31) comprises a processing module and a display screen;
a wrist band (23); wherein
the air pump and the barometric pressure sensor are disposed in the main unit (11); the airbag (34) is packaged in the wrist band.

14. The electronic blood pressure monitor according to claim 11, wherein
the air intake nozzle (36) and the measurement air nozzle (38) are disposed on the same wall of the airbag (34).

## Patentansprüche

1. Luftkissen (34) eines elektronischen Blutdruckmessgeräts, wobei
das Luftkissen (34) eine äußere Wand und mindestens eine Verbindungswand (391, 392, 393, 394) umfasst; wobei die äußere Wand eine erste Wand (37), eine periphere Seitenwand und eine zweite Wand (40) umfasst; wobei die erste Wand (37) und die zweite Wand (40) einander gegenüberliegend angeordnet sind; wobei die periphere Seitenwand mit einer Peripherie der ersten Wand (37) und einer Peripherie der zweiten Wand (40) verbunden ist; wobei die erste Wand (37), die periphere Seitenwand und die zweite Wand (40) eine Luftkammer (S) bilden;
**dadurch gekennzeichnet, dass** sich die mindestens eine Verbindungswand (391, 392, 393, 394) in der Luftkammer (S) befindet;
wobei jede Verbindungswand (391, 392, 393, 394) mit der peripheren Seitenwand und der ersten Wand (37) oder der peripheren Seitenwand und der zweiten Wand (40) verbunden ist; und jede Verbindungswand (391, 392, 393, 394) konfiguriert ist, unter Spannung zu stehen, wenn das Luftkissen (34) in einem ausgedehnten Zustand ist.

2. Luftkissen (34) nach Anspruch 1, wobei
die periphere Seitenwand eine erste Seitenwand (351) und eine zweite Seitenwand (352) umfasst, die einander gegenüberliegen; wobei die mindestens eine Verbindungswand (391, 392, 393, 394) eine erste Verbindungswand (392) und eine zweite Verbindungswand (391) umfasst; wobei die erste Verbindungswand (392) mit der ersten Seitenwand (351) und der ersten Wand (37) verbunden ist; wobei ein Ende der zweiten Verbindungswand (391) mit der zweiten Seitenwand (352) verbunden ist; und ein gegenüberliegendes Ende der zweiten Verbindungswand (391) mit der ersten Wand (37) oder der zweiten Wand (40) verbunden ist.

3. Luftkissen (34) nach Anspruch 1,
wobei die periphere Seitenwand eine erste Seitenwand (351) und eine zweite Seitenwand (352), die einander gegenüberliegen, umfasst; wobei die mindestens eine Verbindungswand (391, 392, 393, 394) eine erste Verbindungswand (392) und eine zweite Verbindungswand (391) umfasst; wobei die erste Verbindungswand (392) mit der ersten Seitenwand (351) und der ersten Wand (37) verbunden ist; und die zweite Verbindungswand (391) mit der zweiten Seitenwand (352) und der ersten Wand (37) verbunden ist.

4. Luftkissen (34) nach Anspruch 3, wobei
die mindestens eine Verbindungswand (391, 392, 393, 394) eine dritte Verbindungswand (393) und eine vierte Verbindungswand (394) umfasst; wobei die dritte Verbindungswand (393) mit der ersten Seitenwand (351) und der zweiten Wand (40) verbunden ist; und die vierte Verbindungswand (394) mir der zweiten Seitenwand (352) und der zweiten Wand (40) verbunden ist.

5. Luftkissen (34) nach Anspruch 1, wobei
jede Verbindungswand (391, 392, 393, 394) ein erstes Ende (391a, 392a), einen mittleren Teil (391b, 392b) und ein zweites Ende (391c, 392c) besitzt, wobei der mittlere Teil (391b, 392b) zwischen dem ersten Ende (391a, 392a) und dem zweiten Ende (391c, 392c) liegt; wobei das erste Ende (391a, 392a) mit der ersten Wand (37) verbunden ist; der mittlere Teil (391b, 392b) mit der peripheren Seitenwand verbunden ist; und das zweite Ende (391c, 392c) mit der zweiten Wand (40) verbunden ist.

6. Luftkissen (34) nach Anspruch 5, wobei jede Verbindungswand (391, 392, 393, 394) Falten besitzt und die Falten konfiguriert sind, in einem gedehnten Zustand zu sein, wenn die Verbindungswand (391, 392, 393, 394) unter Spannung steht.

7. Luftkissen (34) nach einem der Ansprüche 1 bis 6, wobei die mindestens eine Vebindungswand (391, 392, 393, 394) und die äußere Wand verschiedene Kammern (S1-S3) bilden und benachbarte Kammern (S1-S3) kommunizieren.

8. Luftkissen (34) nach Anspruch 7, wobei eine Peripherie jeder Verbindungswand (391, 392, 393, 394) mit der äußeren Wand verbunden ist; wobei jede Verbindungswand (391, 392, 393, 394) mit einem Luftloch (39a) versehen ist; und
benachbarte Kammern (S1-S3) durch das Luftloch (39a) kommunizieren.

9. Luftkissen (34) nach einem der Ansprüche 1 bis 8, wobei
jede Verbindungswand (391, 392, 393, 394) mit einem mittleren Teil der ersten Wand (37) oder einem mittleren Teil der zweiten Wand (40) verbunden ist.

10. Elektronisches Blutdruckmessgerät, das Folgendes umfasst:
eine Luftpumpe;
einen barometrischen Drucksensor; und
das Luftkissen (34) nach einem der Ansprüche 1 bis 9, wobei das Luftkissen (34) eine Luftdüse (38) umfasst, die mit einer Luftkammer (S) verbunden ist und sowohl die Luftpumpe als auch der barometrische Drucksensor mit der Luftkammer (S) durch die Luftdüse (38) kommunizieren.

11. Elektronisches Blutdruckmessgerät nach Anspruch 10, wobei die Luftdüse (38) eine Luftansaugdüse (36) und eine Messluftdüse (38) umfasst, wobei die Luftpumpe mit der Luftkammer (S) durch die Luftansaugdüse (36) kommuniziert und der barometrische Drucksensor mit der Luftkammer (S) durch die Messluftdüse (38) kommuniziert.

12. Elektronisches Blutdruckmessgerät nach Anspruch 10 oder 11, wobei das elektronische Blutdruckmessgerät ein Sphygmomanometer (10), ein Handgelenksblutdruckmessgerät (20) oder ein tragbares Blutdruckmessgerät (30) ist.

13. Elektronisches Blutdruckmessgerät nach Anspruch 10 oder 11, wobei das elektronische Blutdruckmessgerät Folgendes umfasst:
eine Haupteinheit (11, 21, 31), wobei die Haupteinheit (11, 21, 31) ein Verarbeitungsmodul und einen Anzeigebildschirm umfasst;
ein Armband (23); wobei
die Luftpumpe und der barometrische Drucksensor in der Haupteinheit (11) angeordnet sind; wobei das Luftkissen (34) in das Armband gepackt ist.

14. Elektronisches Blutdruckmessgerät nach Anspruch 11, wobei die Luftansaugdüse (36) und die Messluftdüse (38) an derselben Wand des Luftkissens (34) angeordnet sind.

## Revendications

1. Coussin gonflable (34) d'un tensiomètre électronique,
le coussin gonflable (34) comprenant une paroi extérieure et au moins une paroi de liaison (391, 392, 393, 394) ; la paroi extérieure comprenant une première paroi (37), une paroi latérale périphérique, et une deuxième paroi (40) ; la première paroi (37) et la deuxième paroi (40) étant agencées en regard l'une de l'autre ; la paroi latérale périphérique étant reliée à une périphérie de la première paroi (37) et une périphérie de la deuxième paroi (40) ; la première paroi (37), la paroi latérale périphérique, et 1a deuxième paroi (40) formant une cavité d'air (S) ;
**caractérisé en ce que** l'au moins une paroi de liaison (391, 392, 393, 394) est située dans la cavité d'air (S) ; chaque paroi de liaison (391, 392, 393, 394) est reliée à la paroi latérale périphérique et la première paroi (37) ou à la paroi latérale périphérique et la deuxième paroi (40) ; et chaque paroi de liaison (391, 392, 393, 394) est configurée pour se tendre lorsque le coussin gonflable (34) adopte un état dilaté.

2. Coussin gonflable (34) selon la revendication 1,
la paroi latérale périphérique comprenant une première paroi latérale (351) et une deuxième paroi latérale (352) qui sont en regard l'une de l'autre ; l'au moins une paroi de liaison (391, 392, 393, 394) comprenant une première paroi de liaison (392) et une deuxième paroi de liaison (391) ; la première paroi de liaison (392) étant reliée à la première paroi latérale (351) et la première paroi (37) ; une extrémité de la deuxième paroi de liaison (391) étant reliée à la deuxième paroi latérale (352) ; et une extrémité opposée de la deuxième paroi de liaison (391) étant reliée à la première paroi (37) ou la deuxième paroi (40).

3. Coussin gonflable (34) selon la revendication 1,
la paroi latérale périphérique comprenant une première paroi latérale (351) et une deuxième paroi latérale (352) qui sont en regard l'une de l'autre ; l'au moins une paroi de liaison (391, 392, 393, 394) comprenant une première paroi de liaison (392) et une deuxième paroi de liaison (391) ; la première paroi de liaison (392) étant reliée à la première paroi latérale (351) et la première paroi (37) ; et la deuxième paroi de liaison (391) étant reliée à la deuxième paroi latérale (352) et la première paroi (37).

4. Coussin gonflable (34) selon la revendication 3,
l'au moins une paroi de liaison (391, 392, 393, 394) comprenant une troisième paroi de liaison (393) et une quatrième paroi de liaison (394) ; la troisième paroi de liaison (393) étant reliée à la première paroi latérale (351) et la deuxième paroi (40) ; et la quatrième paroi de liaison (394) étant reliée à la deuxième paroi latérale (352) et la deuxième paroi (40).

5. Coussin gonflable (34) selon la revendication 1,
chaque paroi de liaison (391, 392, 393, 394) étant pourvue d'une première extrémité (391a, 392a), d'une partie milieu (391b, 392b), et d'une deuxième extrémité (391c, 392c), la partie milieu (391b, 392b) se situant entre la première extrémité (391a, 392a) et la deuxième extrémité (391c, 392c) ; la première extrémité (391a, 392a) étant reliée à la première paroi (37) ; la partie milieu (391b, 392b) étant reliée à la paroi latérale périphérique ; et la deuxième extrémité (391c, 392c) étant reliée à la deuxième paroi (40).

6. Coussin gonflable (34) selon la revendication 5, chaque paroi de liaison (391, 392, 393, 394) étant pourvue de plis, et les plis étant configurés pour adopter un état étiré lorsque la paroi de liaison (391, 392, 393, 394) se tend.

7. Coussin gonflable (34) selon l'une quelconque des revendications 1 à 6, l'au moins une paroi de liaison (391, 392, 393, 394) et la paroi extérieure formant des cavités (S1-S3) différentes, et des cavités (S1-S3) adjacentes étant en communication.

8. Coussin gonflable (34) selon la revendication 7, une périphérie de chaque paroi de liaison (391, 392, 393, 394) étant reliée à la paroi extérieure ; chaque paroi de liaison (391, 392, 393, 394) étant dotée d'un trou d'air (39a) ; et des cavités (S1-S3) adjacentes étant en communication à travers le trou d'air (39a).

9. Coussin gonflable (34) selon l'une quelconque des revendications 1 à 8,
chaque paroi de liaison (391, 392, 393, 394) étant reliée à une partie milieu de la première paroi (37) ou une partie milieu de la deuxième paroi (40).

10. Tensiomètre électronique, comprenant :
une pompe à air ;
une capteur de pression barométrique ; et
le coussin gonflable (34) selon l'une quelconque des revendications 1 à 9, le coussin gonflable (34) comprenant une buse d'air (38) reliée à une cavité d'air (S), et la pompe à air et le capteur de pression barométrique étant tous deux en communication avec la cavité d'air (S) à travers la buse d'air (38).

11. Tensiomètre électronique selon la revendication 10, la buse d'air (38) comprenant une buse d'admission d'air (36) et une buse d'air de mesure (38), la pompe à air étant en communication avec la cavité d'air (S) à travers la buse d'admission d'air (36), et le capteur de pression barométrique étant en communication avec la cavité d'air (S) à travers la buse d'air de mesure (38).

12. Tensiomètre électronique selon la revendication 10 ou 11, le tensiomètre électronique étant un sphygmomanomètre (10), un tensiomètre au poignet (20), ou un tensiomètre portable sur soi (30).

13. Tensiomètre électronique selon la revendication 10 ou 11, le tensiomètre électronique comprenant :
une unité principale (11, 21, 31), l'unité principale (11, 21, 31) comprenant un module de traitement et un écran d'affichage ;
un bracelet (23) ;
la pompe à air et le capteur de pression barométrique étant agencés dans l'unité principale (11) ; le coussin gonflable (34) étant encapsulé dans le bracelet.

14. Tensiomètre électronique selon la revendication 11,
la buse d'admission d'air (36) et la buse d'air de mesure (38) étant agencées sur la même paroi du coussin gonflable (34).
